Europäisches Patentamt
European Patent Office
Office européen des brevets

(19)

(11) Veröffentlichungsnummer: **0 215 380 A1**

(12) # EUROPÄISCHE PATENTANMELDUNG

(21) Anmeldenummer: **86112215.8**

(22) Anmeldetag: **04.09.86**

(51) Int. Cl.⁴: **C07D 401/12** , C07D 417/12 , C07D 401/14 , C07D 231/08 , C07D 403/12 , A61K 31/415 , A61K 31/41 , A61K 31/425 , A61K 31/505

(30) Priorität: **14.09.85 DE 3532880**

(43) Veröffentlichungstag der Anmeldung: **25.03.87 Patentblatt 87/13**

(84) Benannte Vertragsstaaten: **AT BE CH DE FR GB IT LI NL SE**

(71) Anmelder: **BASF Aktiengesellschaft**
**Carl-Bosch-Strasse 38**
**D-6700 Ludwigshafen(DE)**

(72) Erfinder: **Ostersehlt, Bernd, Dr.**
**Birkenstrasse 18**
**D-6701 Maxdorf(DE)**
Erfinder: **Rieber, Norbert, Dr.**
**Liebfrauenstrasse 1c**
**D-6800 Mannheim 51(DE)**
Erfinder: **Gries, Josef, Dr.**
**Roemerweg 43**
**D-6706 Wachenheim(DE)**

(54) **1,4-Disubstituierte Pyrazolderivate.**

(57) 1,4-Disubstituierte Pyrazolderivate der allgemeinen Formel I

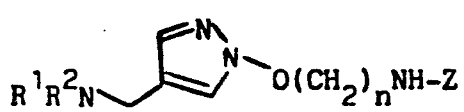
I,

worin R¹, R² jeweils unabhängig voneinander für Wasserstoff, Niedrigalkyl, Benzyl stehen oder zusammen mit dem Stickstoffatom, an das sie gebunden sind, ein Pyrrolidino-, Piperidino-oder Morpholino-Rest sein können, n eine ganze Zahl von 2 bis 5 ist, und Z für einen Rest der Formel

EP 0 215 380 A1

Xerox Copy Centre

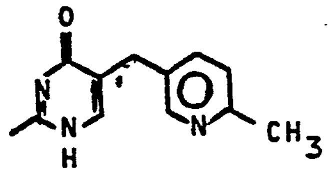, 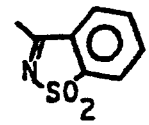 oder $QNR^1R^2$ steht,

wobei $R^1$, $R^2$ dasselbe wie oben bedeuten, Q für

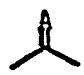, 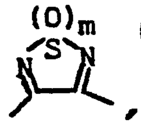, 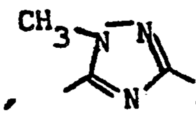, 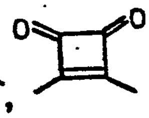,

A für $CHR^3$ oder $NR^3$ mit $R^3$ gleich CN, $NO_2$, $SO_2$Aryl, $SO_2$-Niedrigalkyl steht und m O oder 1 sein kann, sowie deren pharmazeutisch verträglichen Salze, Verfahren zu ihrer Herstellung, ihre Verwendung als Arzneimittel, die Arzneimittel sowie ein Zwischenprodukt der Formel III

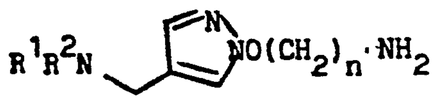 III,

worin R', $R^2$ und n dasselbe wie oben bedeuten, bei der Herstellung der Verbindungen der Formel I.

# 1,4-Disubstituierte Pyrazolderivate

Die Erfindung betrifft neue 1,4-disubstituierte Pyrazolderivate mit selektiver Wirkung auf Histamin-$H_2$-Rezeptoren.

Eine Unterteilung der Histamin-Rezeptoren in zwei Gruppen, nämlich $H_1$-und $H_2$-Rezeptoren ist von Ash und Schild (Brit. J. Pharmacol. Chemother., 27, 427 (1966)) und von Black et al (Nature, 236, 385 (1972)) vorgeschlagen worden. Verbindungen mit Histamin-$H_2$-Rezeptor-blockierender Wirkung sind geeignet zur Behandlung von Krankheitszuständen, die mit einer pathologisch gesteigerten Magensäuresekretion einhergehen, wie z. B. Magen-und Duodenalulcera. Beispiele für solche Verbindungen, die bereits Eingang in die Therapie gefunden haben, sind Cimetidin (US-PS 3 950 333) und Ranitidin (US-PS 4 128 658). Ihre Wirksamkeit ist jedoch relativ schwach, so daß täglich große Dosiseinheiten verabreicht werden müssen. Es besteht daher ein Bedarf an neuen, selektiven Antagonisten des Histamin-$H_2$-Rezeptors mit verbesserter Wirksamkeit. Derartige Verbindungen werden durch die Erfindung zur Verfügung gestellt.

Gegenstand der Erfindung sind 1,4-disubstituierte Pyrazolderivate der allgemeinen Formel I

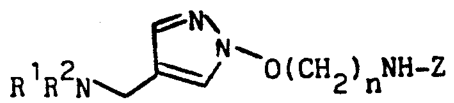 I,

worin $R^1$, $R^2$ jeweils unabhängig voneinander für Wasserstoff, (Niedrigalkyl, Benzyl stehen oder zusammen mit dem Stickstoffatom, an das sie gebunden sind, ein Pyrrolidino-, Piperidino-oder Morpholino-Rest sein können, n eine ganze Zahl von 2 bis 5 ist, und Z für einen Rest der Formel

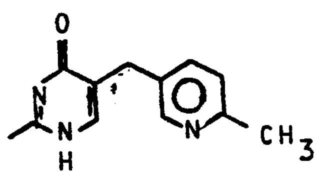 , 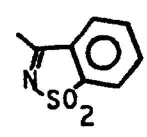 oder $QNR^1R^2$

wobei $R^1$, $R^2$ dasselbe wie oben bedeuten, Q für

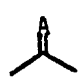 , 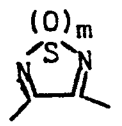 , 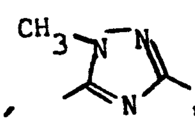 , 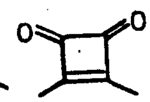 ,

A für $CHR^3$ oder $NR^3$ mit $R^3$ gleich CN, $NO_2$, $SO_2$Aryl, $SO_2$-Niedrigalkyl steht und m O oder 1 sein kann, sowie deren pharmazeutisch verträglichen Salze.

Bei einer bevorzugten Gruppe von Verbindungen hat n den Wert 4.

Bei einer weiteren bevorzugten Gruppe von Verbindungen steht für Z

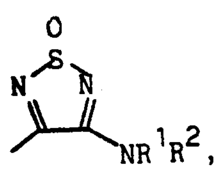

wobei $R^1$ Wasserstoff und $R^2$ Wasserstoff oder Niedrigalkyl bedeuten.

Unter Niedrigalkyl ist stets $C_{1-4}$-Alkyl zu verstehen.

Als erfindungsgemäße Verbindungen der Formel I sind beispielsweise zu nennen:

N-Methyl-N'-[3-(4-piperidinomethylpyrazol-1-oxy)-propylamino]-cyanguanidin,

N-Methyl-N'-[3-(4-dimethylaminomethylpyrazol-1-oxy)propylamino]-cyanguanidin,

N-Methyl-N'[4-(4-piperidinomethylpyrazol-1-oxy)-butylamino]-cyanguanidin,

N-Methyl-N'[4-(4-dimethylaminomethylpyrazol-1-oxy)butylamino]-cyanguanidin,

1-Methylamino-1-[3-(4-N,N-dimethylaminomethylpyrazol-1-oxy)propylamino]-2-nitroethylen,

1-Methylamino-1-[3-(4-piperidinomethylpyrazol-1-oxy)propylamino]-2-nitroethylen,

1-Methylamino-1-[4-(4-N,N-dimethylaminomethylpyrazol-1-oxy)butylamino]-2-nitroethylen,

1-Methylamino-1-[4-(4-piperidinomethylpyrazol-1-oxy)butylamino]-2-nitroethylen,

2-[3-(4-Piperidinomethylpyrazol-1-oxy)-propylamino]-5-(2-methyl-5-pyridylmethyl)-pyrimidon-4,

2-[4-(4-Piperidinomethylpyrazol-1-oxy)butylamino]-5-(2-methyl-5-pyridylmethyl)-pyrimidon-4,

3-Amino-4[3-(4-piperidinomethylpyrazol-1-oxy)-propylamino]-cyclobutendion-1,2,

3-Amino-4[4-(4-piperidinomethylpyrazol-1-oxy)-butylamino]-cyclobutendion-1,2,

3-Methylamino-4[3-(4-piperidinomethylpyrazol-1-oxy)propylamino]-cyclobutendion-1,2,

3-Methylamino-4[4-(4-piperidinomethylpyrazol-1-ox)butylamino]-cyclobutendion-1,2,

1-Methyl-3-amino-4-[3-(4-piperidinomethylpyrazol-1-oxy)propylamino]-1,2,4-triazol,

1-Methyl-3-amino-4[4-(4-piperidinomethylpyrazol-1-oxy)butylamino]-1,2,4-triazol,

1-Methyl-3-amino-4[5-(4-piperidinomethylpyrazol-1-oxy)pentylamino]-1,2,4-triazol,

1-Methyl-3-amino-4[3-(4-dimethylaminomethylpyrazol-1-oxy)propylamino]-1,2,4-triazol,

1-Methyl-3-amino-4[4-(4-dimethylaminomethylpyrazol-1-oxy)butylamino]-1,2,4-triazol,

4-Methylamino-3[3-(4-piperidinomethylpyrazol-1-oxy)propylamino]-1,2,5-thiadiazol-1-oxid,

4-Methylamino-3-[4-(4-piperidinomethylpyrazol-1-oxy)butylamino]-1,2,5-thiadiazol-1-oxid,

4-Methylamino-3-[3-(4-dimethylaminomethylpyrazol-1-oxy)propylamino]-1,2,5-thiadiazol-1-oxid,

4-Methylamino-3-[4-(4-dimethylaminomethylpyrazol-1-oxy)butylamino]-1,2,5-thiadiazol-1-oxid,

4-Amino-3[3-(4-dimethylaminomethylpyrazol-1-oxy)propylamino]-1,2,5-thiadiazol-1-oxid,

4-Amino-3[4-(4-dimethylaminomethylpyrazol-1-oxy)butylamino]-1,2,5-thiadiazol-1-oxid,

4-Amino-3[3-(4-piperidinomethylpyrazol-1-oxy)-propylamino]-1,2,5-thiadiazol-1-oxid,

4-Amino-3[4-(4(4-piperidinomethylpyrazol-1-oxy)-butylamino]-1,2,5-thiadiazol-1-oxid,

4-Amino-3[5-(4-piperidinomethylpyrazol-1-oxy)-pentylamino]-1,2,5-thiadiazol-1-oxid,

4-Amino-3[4-(4-di-n-butylaminomethylpyrazol-1-oxy)butylamino]-1,2,5-thiadiazol-1-oxid,

4-Amino-3[4-(4--pyrrolidinomethylpyrazol-1-oxy)-butylamino]-1,2,5-thiadiazol-1-oxid,

4-Amino-3[4-(4-morpholinomethylpyrazol-1-oxy)-butylamino]-1,2,5-thiadiazol-1-oxid,

4-Amino-3[4-(4-N-methyl-N-benzylaminomethylpyrazol-1-oxy)butylamino-1,2,5-thiadiazol-1-oxid,

3-[4-(4-dimethylaminomethylpyrazol-1-oxy)-butylamino]benzisothiazol-1,1-dioxid,

3-[4(4-piperidinomethylpyrazol-1-oxy)butylamino]-benzisothiazol-1,1-dioxid,

4-Amino-3-[4-(4-dimethylaminomethylpyrazol-1-oxy)butylamino]-1,2,5-thiadiazol,

4-Amino-3-[4-(4-piperidinomethylpyrazol-1-oxy)-butylamino]-1,2,5-thiadiazol.

Als physiologisch verträgliche organische oder anorganische Säuren kommen zur Salzbildung beispielsweise in Betracht: Salzsäure, Bromwasserstoffsäure, Phosphorsäure, Schwefelsäure, Maleinsäure, Fumarsäure, Milchsäure, Weinsäure,

Apfelsäure, Zitronensäure, Salicylsäure, Adipinsäure oder Benzoesäure. Weitere Säuren können aus Fortschritte der Arzneimittelforschung, Band 10, Seiten 224 bis 225, Birkhauser-Verlag, Basel und Stuttgart, 1966, entnommen werden.

Die Erfindung betrifft weiter Verfahren zur Herstellung von Verbindungen der Formel I, welche darin bestehen, daß man

a) zur Herstellung von Verbindungen der Formel I mit Z gleich

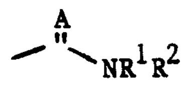

eine Thioverbindung der Formel II

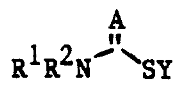

II,

in der $R^1$, $R^2$ und A dasselbe wie oben und Y Niedrigalkyl bedeuten, mit einem Amin der Formel III

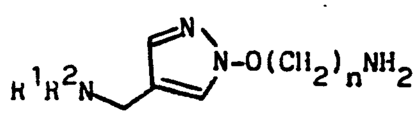

III,

in der $R^1$, $R^2$ und n die angegebene Bedeutung haben, umsetzt oder eine Verbindung der Formel III mit einer Verbindung der Formel IV oder V

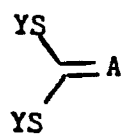

IV,

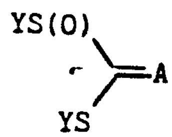

V,

in denen A und Y dasselbe wie oben bedeuten, umsetzt und die resultierende Verbindung der Formel VI

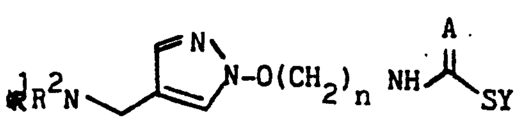

VI,

in der $R^1$, $R^2$, n und A die angegebene Bedeutung haben, mit einer Aminoverbindung der Formel VII

$HNR^1R^2$ VII,

worin $R^1$, $R^2$ dasselbe wie oben bedeuten, reagieren läßt oder

b) zur Herstellung von Verbindungen der Formel I mit Z gleich

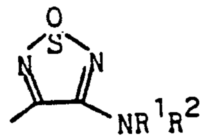

ein Thiadiazolderivat der Formel VIII

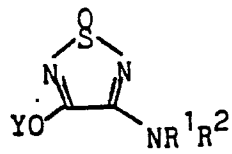 VIII,

in der R', R² und Y dasselbe wie oben bedeuten, mit einer Verbindung der Formel III umsetzt oder eine Verbindung der Formel III mit einer Verbindung der Formel IX

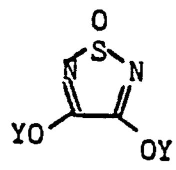 IX,

in der Y Niedrigalkyl bedeutet, umsetzt und die resultierende Verbindung der Formel X

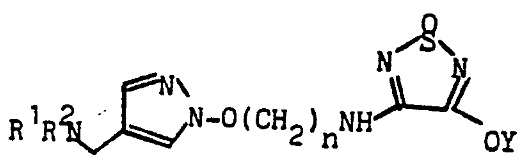 X

mit einer Aminoverbindung der Formel VII umsetzt oder

c) zur Herstellung von Verbindungen mit Z gleich

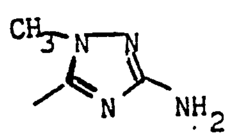

ein Hydrazinderivat der Formel XI

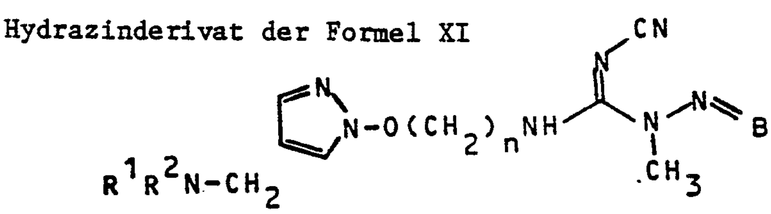 XI,

in der Y Niedrigalkyl und B eine Schutzgruppe, beispielsweise =CH-Ø, bedeuten, mit einer Verbindung der Formel III umsetzt und das Reaktionsprodukt in Gegenwart von Säuren katalytisch spaltet und cyclisiert oder

d) zur Herstellung von Verbindungen der Formel I mit Z gleich

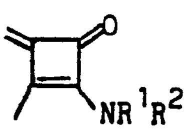

ein Quadratsäurederivat der Formel XII

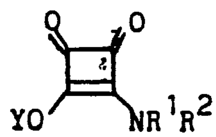

XII,

in der $R^1$, $R^2$ und Y dasselbe wie oben bedeuten, mit einer Verbindung der Formel III umsetzt oder ein Quadratsäurederivat der Formel XIII

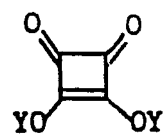

XIII,

in der Y (Niedrig)alkyl bedeutet, mit einer Verbindung der Formel III umsetzt und das resultierende Produkt der Formel XIV

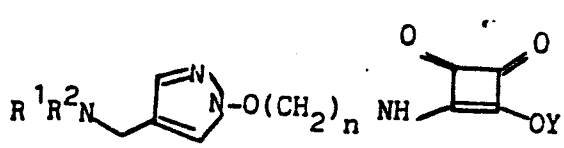

XIV,

in der $R^1$, $R^2$, Y und n dasselbe wie oben bedeuten, mit einem Amin der Formel VII umsetzt oder

e) zur Herstellung von Verbindungen der Formel I mit Z gleich

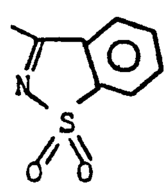

eine Verbindung der Formel XV

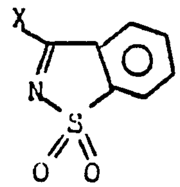

XV,

worin X eine geeignete Austrittsgruppe wie Halogen, OY, SY mit Y gleich Niedrigalkyl bedeutet, mit einer Verbindung der Formel III umsetzt, oder

f) zur Herstellung von Verbindungen der Formel I mit Z gleich

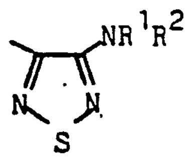

eine Verbindung der Formel I mit Z gleich

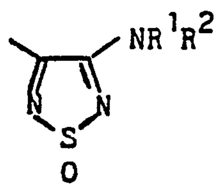

mit einer starken Säure behandelt und das Produkt der Formel XVI

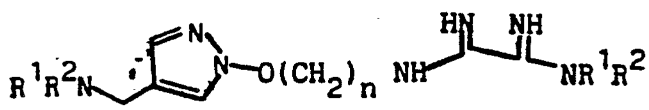

XVI

mit einer Verbindung der Formel XVII

L-S-L XVII,

in der L eine geeignete Austrittsgruppe, vorzugsweise Phthalimido-bedeutet, umsetzt, und die so erhaltenen Verbindungen in ihre Salze mit physiologisch verträglichen Säuren überführt, oder

g) zur Herstellung von Verbindungen der Formel I mit Z gleich

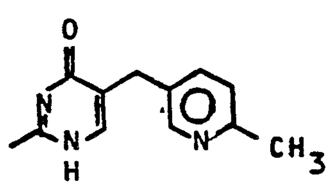

eine Verbindung der Formel III mit einer Verbindung der Formel

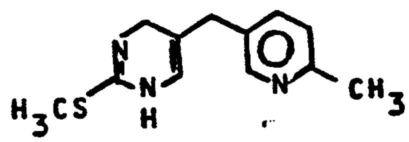

umsetzt.

Die Umsetzung gemäß a) wird bei Raumtemperatur bis 140°C, zweckmäßig bei 50 bis 120 °C, durchgeführt. Sie kann unter Atmosphärendruck oder in einem geschlossenen Gefäß unter erhöhtem Druck, gegebenenfalls unter Erwärmung auf den angegebenen Temperaturbereich vorgenommen werden. Besonders gut gelingt sie, wenn Y eine Methylgruppe bedeutet.

Die Ausgangsverbindungen können direkt, d. h. ohne Zugabe eines Verdünnungsmittels, umgesetzt werden. Zweckmäßigerweise wird die Umsetzung jedoch in Gegenwart eines inerten Verdünnnungs- oder Lösungsmittels, beispielsweise eines niederen Alkohols mit 1 bis 4 C-Atomen, wie Methanol, Ethanol oder Propanol, eines niederen gesättigten Dialkylethers, Dialkylglykolethers oder cyclischen Ethers, wie Diethylether, 1,2-Dimethoxyether, Tetrahydrofuran oder Dioxan, eines Dialkylformamids, wie Dimethyl-oder Diethylformamid, von Dimethylsulfoxid, Acetonitril oder in Gegenwart von Wasser oder in Mischungen der genannten Lösungsmittel, durchgeführt. Am geeignetsten sind niedere Alkohole, vorzugsweise Methanol und Ethanol sowie Wasser.

Die vollständige Umsetzung hängt von der Reaktionstemperatur ab und ist im allgemeinen innerhalb von 2 bis 25 Stunden beendet. Das Reaktionsprodukt kann in an sich bekannter Weise gewonnen werden, z. B. durch Filtration oder Abdestillieren des Verdünnungs-oder Lösungsmittels aus dem Reaktionsgemisch. Eine Reinigung der erhaltenen Verbindungen erfolgt in üblicher Weise, beispielsweise durch Umkristallisation aus einem Lösungsmittel, Überführen in das Salz einer physiologisch verträglichen Säure oder durch Säulenchromatographie.

Die Umsetzungen b) -e) und g) werden in Gegenwart eines Lösungsmittels, beispielsweise eines niederen Alkohols mit 1 bis 4 C-Atomen, vorzugsweise Methanol, Ethanol oder Isopropanol, oder eines niederen gesättigten Dialkylethers oder cyclischen Ethers wie Diethylether, Methyl-tert.-butylether, Tetrahydrofuran, oder eines niederen Chlorkohlenwasserstoffes wie Methylenchlorid, Chloroform, oder eines Dialkylformamids, wie Dimethyl-oder Diethylformamid, von Dimethylsulfoxid oder von Wasser durchgeführt. Dem Reaktionsgemisch kann, wenn erforderlich, eine anorganische oder organische Base, z. B. ein Alkali-oder Erdalkalimetallcarbonat oder ein tertiäres Amin wie Triethylamin oder Pyridin, zugesetzt werden. Die Reaktion erfolgt bei Temperaturen von 0 bis 100 °C unter Normaldruck. Die Gewinnung der Reaktionsprodukte kann wie bei a) beschrieben erfolgen. Das Verfahren f) ist bekannt und wird im Prinzip beispielsweise in der DE-OS 3 211 281 beschrieben.

Für die Verfahren zur Herstellung der erfindungsgemäßen Verbindungen werden primäre Amine der Formel III verwendet. Diese Amine sind neue Verbindungen. Die Erfindung umfaßt auch diese Verbindungen und ihre Säureadditionssalze. Die Amine der Formel III können hergestellt werden, indem man

h) 1-Hydroxypyrazol mit einer Phthalimidoverbindung der Formel XVIII

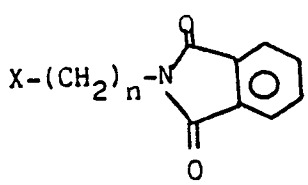

XVIII,

wobei n dasselbe wie oben und X eine Austrittsgruppe, z. B. Halogen oder Sulfonyloxy, wie Mesyl, Tosyl, sein kann, unter Verwendung einer Base, wie eines Alkalimetallcarbonates, -hydroxids oder -alkoxids oder Natriumhydrids, in einem inerten Lösungsmittel, wie Toluol, Xylol, Tetrahydrofuran oder Dimethylformamid, umsetzt.

Das Reaktionsprodukt der Formel IXX

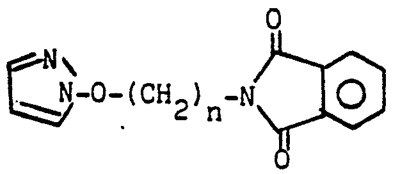

IXX

wird dann nach Art einer Mannich-Reaktion, vorzugsweise mit einem Reagens der Formel XX

$(CH_2=NR^1R^2)^{\oplus}Cl^{\ominus}$ XX,

wobei $R^1$, $R^2$ dasselbe wie oben bedeuten, umgesetzt. Die Reaktion wird in einem inerten Lösungsmittel, vorzugsweise Acetonitril oder Dimethylformamid bei erhöhter Temperatur, vorzugsweise zwischen 50 °C und 150 °C, durchgeführt.

Schließlich kann in der resultierenden Verbindung der Formel XXI

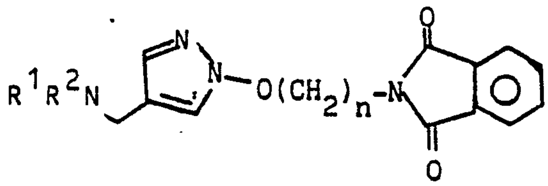

XXI

die Phthalimidoschutzgruppe durch ein geeignetes Reagens wie ein Alkalimetallhydroxid, oder ein primäres Amin wie Methylamin oder Ethanolamin, oder Hydrazin abgespalten werden;

oder indem man

i) 1-Hydroxypyrazol-4-carbonsäuremethylester mit einem ω-substituierten Aldehydacetal der Formel XXII

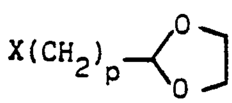

XXII,

wobei X dasselbe wie oben bedeutet und p den Wert n -1 hat, umsetzt. Das Verfahren entspricht dem bei der Umsetzung von XVIII beschriebenen. Im Reaktionsprodukt der Formel XXIII wird die Estergruppe nach Hydrolyse zur Carbonsäure mit einem Amin der Formel VII ins Amid überführt. Dazu wird die Carbonsäure zwischenzeitlich in ein aktiviertes Derivat, z. B. ein Säurehalogenid, ein Säureanhydrid oder einen aktivierten Ester umgewandelt.

Aus dem Zwischenprodukt der Formel XXIV wird mit verdünnter wäßriger Mineralsäure in bekannter Weise der Aldehyd XXV gebildet und dieser mit Hydroxylamin ins Oxim XXVI überführt.

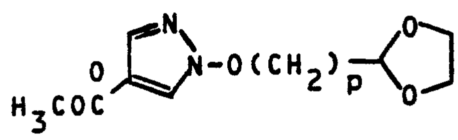

XXIII

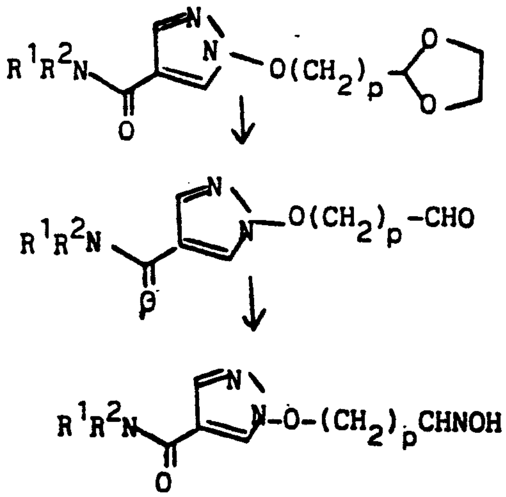

XXIV

XXV

XXVI

Das Oxim XXVI kann mit geeigneten Reduktionsmitteln, z. B. komplexen Metallhydriden wie Lithiumaluminiumhydrid ins Amin III umgewandelt werden.

Die Ausgangssubstanzen der Formeln II, IV, V, VIII, IX, XI, XII, XIII, XV, XVII, XVIII, XX und XXII sind literaturbekannt oder lassen sich nach bekannten Verfahren herstellen.

Schließlich betrifft die Erfindung Arzneimittel, welche neben üblichen Träger-und Verdünnungsmitteln eine Verbindung der Formel I oder ihr physiologisch verträgliches Salz als Wirkstoff enthalten.

Die erfindungsgemäßen Verbindungen und ihre physiologisch verträglichen Säureadditionssalze weisen wertvolle pharmakologische Eigenschaften auf. Sie eignen sich als Histamin-Antagonisten mit hoher und spezifischer Wirkung auf Histamin-$H_2$-Rezeptoren pharmakotherapeutisch besonders zur Behandlung von Krankheitszuständen, die mit einer pathologisch gesteigerten Sekretion von Magensäure einhergehen, z. B. Magen-oder Duodenalulcera.

Die erfindungsgemäßen Verbindungen können in üblicher Weise, oral oder intravenös, verabfolgt werden. Die Dosierung hängt vom Alter, Zustand und Gewicht des Patienten sowie von der Applikationsart ab. In der Regel beträgt die tägliche Wirkstoffdosis zwischen etwa 0,1 und 20 mg/kg Körpergewicht bei oraler Gabe und zwischen 0,01 und 10 mg/kg Körpergewicht bei intravenöser Gabe. In besonderen Fällen kann es jedoch erforderlich sein, die Dosen zu steigern.

Die neuen Verbindungen können in den üblichen galenischen Applikationsformen fest oder flüssig angewendet werden, wie Tabletten, Kapseln, Pulver, Granulate, Dragees oder Lösungen. Diese werden in üblicher Weise hergestellt. Die Wirkstoffe können dabei mit den gebräuchlichen galenischen Hilfsmitteln wie Talkum, Gummiarabikum, Saccharose, Lactose, Getreide-oder Maisstärke, Kartoffelmehl, Magnesiumstearat, Alginaten, Gummitragacanth, Carraghenate, Polyvinylalkohol, Polyvinylpyrrolidon, wäßrigen oder nicht wäßrigen Trägern, Netzmitteln, Dispergiermitteln, Emulgatoren und/oder Konservierungsmitteln verarbeitet werden (vgl. Hagers Handbuch der Pharmazeut. Praxis, 4. Aufl., Springer-Verl. Berlin -Heidelberg -New York 1967-1980, Bd. VII A u. B). Die so erhaltenen Präparate enthalten den Wirkstoff normalerweise in einer Menge von 0,1 bis 99 Gewichtsprozenten.

Die folgenden Beispiele sollen die Erfindung näher erläutern (die NMR-Daten sind in δ-Werten - (ppm) angegeben).

A. Herstellung der Ausgangsverbindungen und Zwischenprodukte

Beispiel A

1-Hydroxypyrazol-4-carbonsäuremethylester

90 g 1-Hydroxypyrazol-4-carbonsäure (zur Herstellung vgl. deutsche Parallelanmeldung P 35 32 879.7) werden in 500 ml Methanol suspendiert und mit 10 ml konzentrierter Schwefelsäure versetzt. Anschließend wird 12 h unter Rückfluß gekocht. Das Methanol wird im Vakuum abdestilliert, der feste Rückstand in 400 ml Wasser verrührt, mit Natronlauge auf pH 3 gestellt, abgesaugt, mit Wasser gewaschen. Die Mutterlauge wird eingeengt und eine weitere Fraktion Kristallisat

gewonnen. Die vereinigten Produkte werden im Vakuum getrocknet und ergeben 91 g (91 % der Theorie) 1-Hydroxypyrazol-4-carbonsäuremethylester vom Fp. 175 -181 °C. NMR - (DMSO-$D_6$): 8,35 s [1]; 7,82 s [1]; 3,80 s [3].

Beispiel B

a) 1-[3-(2-dioxolanyl)propoxy]-pyrazol-4-carbonsäuremethylester

21,8 g Natriumhydrid (80prozentige Suspension in Mineralöl) wird in 1,5 l trockenes Dimethylformamid eingetragen. Unter Rühren werden 90 g 1-Hydroxypyrazol-4-carbonsäuremethylester portionsweise zugegeben. Nach beendeter Wasserstoffentwicklung wird 30 Min. nachge rührt. Dann werden 95,5 g 2(3-Chlorpropyl)-1,3-dioxolan zugetropft und die Mischung 10 h bei 120 °C gehalten. Anschließend wird das Dimethylformamid im Vakuum abdestilliert und der Rückstand in Methyl-tert.-butylester aufgenommen, mit Wasser gewaschen, gekocht und eingeengt. Es verbleiben 151 g leicht gefärbtes Öl, das durchkristallisiert, Fp. 40 -42 °C. NMR ($CDCl_3$): 7,82 s [1]; 7,70 s [1]; 4,50 t [1]; 4,39 t [2]; 3,85 -3,95 m [4]; 3,80 s [3]; 1,75 -1,95 m [4].

b) 1-[3-(2-dioxolanyl)propoxy]-pyrazol-4-carbonsäure

151 g des obigen Produktes aus a) werden mit 650 ml 2 n Natronlauge bei 90 °C so lange gerührt, bis eine klare Lösung entstanden ist. Nach Abkühlen wird mit 200 ml Methylenchlorid gewaschen, die wäßrige Phase mit konzentrierter Salzsäure angesäuert, und mehrmals mit Methylenchlorid extrahiert. Der Extrakt wird getrocknet und eingeengt. Es verbleibt ein farbloser Rückstand, der spontan kristallisiert. Ausbeute:129 g (88 % der Theorie bezogen auf 1-Hydroxypyrazol-4-carbonsäuremethylester). Fp.: 85 °C

NMR ($CDCl_3$): 7,92 s [1]; 7,75 s [1]; 4,52 t [2]; 4,35 t [2]; 3,80 -4,05 m [4]; 1,75 -2,00 m [4].

Beispiel C

a) 1-[3-(2-dioxolanyl)propoxy]-4-(N-piperidylcarbonyl)pyrazol

14,5 g Pivalinsäurechlorid werden in 300 ml Methylenchlorid gelöst. Bei -30 °C wird eine Lösung von 12,2 g Triethylamin und 29 g 1-[3-(2-dioxolanyl)propoxy)-pyrazol-4-carbonsäure in 100 ml Methylenchlorid zugetropft. Nach beendeter Zugabe wird auf 20 °C erwärmt und 11,2 g Piperidin zugegeben. Zur Aufarbeitung wird mit verdünnter Salzsäure und verdünnter Natronlauge gewaschen und eingeengt. Es verbleiben 35 g leicht gefärbtes Öl.

NMR ($CDCl_3$): 7,60 s [1]; 7,38 s [1]; 4,50 t [1]; 4,35 t [2]; 3,80 -4,50 m [4]; 3,50 -3,70 m [4]; 1,45 -1,95 m [10].

In analoger Weise wurden hergestellt:

b) 1-[3-(2-Dioxolanyl)propoxy]-4-(N-pyrrolidinyl)-carbonyl-pyrazol

NMR ($CDCl_3$): 7,69 s [1]; 7,48 s [1]; 4,82 t [1]; 4,30 t [2]; 3,75 -3,95 m [4]; 3,45 -3,70 m [4]; 1,70 -2,10 m [8].

c) 1-[3-(2-Dioxolanyl)propoxy]-4-(N-morpholinyl)-carbonyl-pyrazol

NMR ($CDCl_3$): 7,61 s [1]; 7,36 s [1]; 4,86 t [1]; 4,33 t [2]; 3,80 -4,00 m [4]; 3,68 s [8]; 1,75 -1,95 m [4].

d) 1-[3-(2-Dioxolanyl)propoxy]-pyrazol-4-carbonsäure-N,N-di-n-butylamid

NMR ($CDCl_3$): 7,54 s [1]; 7,30 s [1]; 4,82 t [1]; 4,30 t [2]; 3,70 -3,95 m [4]; 3,38 t [4]; 1,75 -1,90 m [4]; 1,20 -1,70 m [8]; 0,90 t [6].

e) 1-[3-(2-Dioxolanyl)propoxy)-pyrazol-4-carbonsäure-N-methyl-N-benzylamid

NMR ($CDCl_3$): 7,61 s [1]; 7,40 s [1]; 7,15 -7,30 m [5]; 4,83 t [1]; 4,63 s [2]; 4,31 t [2]; 3,75 -3,95 m [4]; 3,25 s [3]; 1,80 -1,95 m [4].

Beispiel D

1-(4-Oxobutoxy)-4-(N-piperidinyl)carbonyl-pyrazol

a) 40 g 1-[3-(2-dioxolanyl)propoxy]-4-(N-piperidinyl)carbonyl-pyrazol werden in einer Mischung aus 150 ml Tetrahydrofuran, 200 ml Wasser und 4 ml konzentrierter Schwefelsäure 8 h am Rückfluß gekocht. Nach Abkühlen wird mit Kochsalz gesättigt, die Phasen getrennt und die wäßrige Phase nochmals mit Tetrahydrofuran extrahiert. Die vereinigten organischen Phasen werden eingeengt,

der Rückstand mit Methylenchlorid aufgenommen und mit Wasser gewaschen. Nach Abdampfen des Lösungsmittel verbleiben 33 g der Titelverbindung als viskoses Öl.

NMR: 9,83 s [1]; 7,67 s [1]; 7,43 s [1]; 4,38 t [2]; 3,50 -3,80 m [4]; 2,71 t [2]; 1,50 -2,30 m [8].

Auf analoge Weise wurden hergestellt:

b) 1-(4-Oxobutoxy)-4-(N-pyrrolidinyl)carbonyl-pyrazol

NMR ($CDCl_3$): 9,85 s [1]; 7,85 s [1], 7,65 s [1]; 4,40 t [2]; 3,50 -3,80 m [4]; 2,75 t [2]; 1,70 -2,30 m [6].

c) 1-(4-Oxobutoxy)-4-(N-morpholinyl)carbonyl-pyrazol

NMR ($CDCl_3$): 9,81 s [1]; 7,78 s [1]; 7,49 s [1]; 4,40 t [2]; 3,60 -3,90 m [8]; 2,75 t [2]; 1,90 -2,30 m [2].

d) 1-(4-Oxobutoxy)-pyrazol-4-carbonsäure-N,N-di-n-butylamid

NMR ($CDCl_3$): 9,75 s [1]; 7,68 s [1]; 7,39 s [1]; 4,30 t [2]; 3,40 t [4]; 2,65 t [2]; 1,20 -2,20 m [10; 0,90 t [6].

e) 1-(4-Oxobutoxy)-pyrazol-4-carbonsäure-N-methyl-N-benzylamid

NMR ($CDCl_3$): 9,81 s [1]; 7,75 s [1]; 7,52 s [1]; 7,30 -7,40 m [5]; 4,77 s [2]; 4,42 t [2]; 3,15 s [3]; 2,75 t [2]; 1,85 -2,30 m [2].

Beispiel E

4-(4-Piperidinomethylpyrazol-1-oxy)butylamin

a) 26 g 1-(1-(4-Oxobutoxy)-4-(N-piperidinyl)-carbonyl-pyrazol, 9 ml Pyridin und 7,3 g Hydroxylammoniumchlorid werden in 250 ml Ethanol gelöst und 5 h bei 25 °C stehen gelassen. Anschließend wird das Lösungsmittel abgedampft, der Rückstand in Methylenchlorid aufgenommen, mit verdünnter Salzsäure und Wasser gewaschen und die resultierende klare Lösung eingeengt. Es verbleiben 27 g eines zähen Öls, die in 40 ml trockenem Tetrahydrofuran gelöst werden. Diese Lösung wird zu einer Suspension von 12 g Lithiumaluminiumhydrid in 350 ml trockenem Tetrahydrofuran getropft. Die Mischung wird 2 h am Rückfluß gekocht, nach Abkühlen wird hydrolysiert, vom Aluminiumhydroxid abgesaugt und das Filtrat eingedampft. Der Rückstand wird mit verdünnter Salzsäure aufgenommen. Die saure wäßrige Lösung wird mit Methylenchlorid gewaschen, alkalisch gestellt und wieder mit Methylenchlorid extrahiert. Nach Abdestillieren des Lösungsmittels verbleiben 12 g schwach gefärbtes Öl.

NMR ($CDCl_3$): 7,35 s [1]; 7,22 s [1]; 4,35 t [2]; 3,40 s [2]; 2,75 t [2]; 2,30 -2,55 m [4]; 1,40 -1,85 m [10]

Auf analoge Weise wurden hergestellt:

b) 4-(4-Pyrrolidinomethylpyrazol-1-oxy)butylamin

NMR ($CDCl_3$): 7,30 s [1]; 7,16 s [1]; 4,30 t [2]; 3,50 s [2]; 2,75 t [2]; 2,40 -2,65 m [4]; 1,60 -2,00 m [8].

c) 4-(4-Morpholinomethylpyrazol-1-oxy)butylamin

NMR($CDCl_3$): 7,35 s [1]; 7,23 s [1]; 4,35 t [2]; 3,60 -3,83 m [4]; 3,40 s [2]; 3,79 t [2]; 2,35 - 2,55 m [4]; 1,60 -1,80 m [4].

d) 4-[4-(N-Methyl-N-benzylaminomethyl)pyrazol-1-oxy]butylamin

NMR ($CDCl_3$): 7,32 s [6]; 7,22 s [1]; 4,32 t [2]; 3,51 s [2]; 3,45 s [2]; 2,76 t [2]; 2,20 s [3]; 1,50 - 1,85 m [4].

e) 4-[4-(N,N-Di-n-butylaminomethyl)pyrazol-1-oxy]-butylamin

NMR ($CDCl_3$): 7,25 s [1]; 7,13 s [1]; 4,31 t [2]; 3,48 s [2]; 2,65 -2,95 m [2]; 2,20 -2,50 m [4]; 1,20 -1,80 m [12]; 0,90 t [6].

Beispiel F

a) 3-(Pyrazol-1-oxy)-propylphthalimid

26 g 1-Hydroxypyrazol wird zu einer Suspension von 10 g Natriumhydrid (80prozentig in Öl) in 500 ml Xylol gegeben. Nach 1 h Rühren werden 84 g 3-Brompropylphthalimid zugefügt und die Mischung 2 h am Rückfluß gekocht. Nach Abkühlen wird filtriert, das Filtrat eingeengt, der kristalline Rückstand in Diethylether suspendiert und abgesaugt. Es werden 85 g der Titelverbindung vom Fp. 116 -118 erhalten.

NMR ($CDCl_3$): 7,65 -7,85 m [4]; 7,45 d [1]; 7,20 d [1]; 6,15 t [1]; 4,38 t [2]; 3,90 t [2]; 1,90 -2,40 m [2].

In analoger Weise wurden hergestellt:

4-(Pyrazol-1-oxy)-butylphthalimid

NMR ($CDCl_3$): 7,55 -7,90 m [4]; 7,30 d [1]; 7,18 d [1]; 6,10 t [1]; 4,35 t [2]; 3,75 t [2]; 1,60 - 2,00 m [4].

c) 5-(Pyrazol-1-oxy)-pentylphthalimid

NMR ($CDCl_3$): 7,55 -7,90 m [4]; 7,30 d [1]; 7,20 d [1]; 6,10 t [1]; 4,25 t [2]; 3,65 t [2]; 1,50 - 1,90 m [6].

Beispiel G

a) 3-(4-N,N-Dimethylaminomethylpyrazol-1-oxy)-propylphthalimid

8,5 g 3-Pyrazol-1-oxy-propylphthalimid und 5 g N,N-Dimethylmethylenammoniumchlorid werden in 150 ml Acetonitril 70 h am Rückfluß gekocht. Anschließend wird eingeengt, der Rückstand in Wasser aufgenommen, mit Methylenchlorid gewaschen. Die wäßrige Phase wird alkalisch gestellt und mit Methylenchlorid extrahiert. Nach Abziehen des Lösungsmittels erhält man 68 g der Titelverbindung.

NMR ($CDCl_3$): 7,60 -7,80 m [4]; 7,30 s [1]; 7,08 s [1]; 4,30 t [2]; 3,85 t [2]; 3,27 s [2]; 2,20 s - [3]; 2,00 -2,20 m [2].

Auf analoge Weise wurden hergestellt:

b) 3-(4-Piperidinomethylpyrazol-1-oxy)-propylphthalimid

NMR ($CDCl_3$): 7,62 -7,78 m [4]; 7,81 s [1]; 7,10 s [1]; 4,32 t [2]; 3,84 t [2]; 3,30 s [2]; 2,21 -2,48 m [4]; 2,00 -2,20 m [2]; 1,35 -1,70 m [6].

c) 4-(4-Piperidinomethylpyrazol-1-oxy)-butylphthalimid

NMR ($CDCl_3$): 7,60 -7,95 m [4]; 7,26 s [1]; 7,11 s [1]; 4,30 t [2]; 3,71 t [2]; 3,30 s [2]; 2,20 -2,50 m [4]; 1,35 -2,00 m [10].

d) 5-(4-Piperidinomethylpyrazol-1-oxy)-pentylphthalimid

NMR ($CDCl_3$): 7,55 -7,90 m [4]; 7,22 s [1]; 7,10 [1]; 4,25 t [2]; 3,68 t [2]; 3,31 s [2]; 2,20 - 2,50 m [4]; 1,30 -1,90 m [12].

Beispiel H

a) 3-(4-N,N-Dimethylaminomethylpyrazol-1-oxy)-propylamin

6,8 g 3-(4-N,N-Dimethylaminomethylpyrazol-1-oxy)propylphthalimid wurden in 40 ml Ethanol gelöst, mit 1,2 g Hydrazinhydrat versetzt und 5 h am Rückfluß gekocht. Nach Abkühlen wird mit konzentrierter Salzsäure angesäuert, kurz aufgekocht, wieder abgekühlt und abgesaugt. Das Filtrat wird eingeengt, der Rückstand in Natronlauge gelöst und mit Methylenchlorid extrahiert. Nach Vertreiben des Lösungsmittels verbleiben 2,8 g der Titelverbindung.

NMR ($CDCl_3$): 7,35 s [1]; 7,15 s [1]; 4,38 t [2]; 3,30 s [2]; 2,85 t [2]; 2,19 s [6]; 1,70 -1,90 m [2].

Auf analoge Weise wurden hergestellt:

b) 3-(4-Piperidinomethylpyrazol-1-oxy)propylamin

NMR ($CDCl_3$): 7,34 s [1]; 7,20 s [1]; 4,35 t [2]; 3,38 s [2]; 2,83 t [2]; 2,30 -2,55 m [4]; 1,40 -1,90 m [8].

c) 4-(4-Piperidinomethylpyrazol-1-oxy)butylamin

NMR: siehe Beispiel Ea).

d) 5-(4-Piperidinomethylpyrazol-1-oxy)pentylamin

NMR ($CDCl_3$): 7,36 s [1]; 7,21 s [1]; 4,30 t [2]; 3,41 s [2]; 2,75 t [2]; 2,30 -2,55 m [4]; 1,40 -1,90 m [12].

Herstellung der erfindungsgemäßen Verbindungen:

Beispiel 1

2-[3-(4-Piperidinomethylpyrazol-1-oxy)-propylamino]-5-(2-methylpyrid-5-ylmethyl)1H-pyrimidon-4

3,5 g 3-(4-Piperidinomethylpyrazol-1-oxy)-propylamin und 3,6 g 2-Methylthio-5-(2-methylpyrid-5-ylmethyl-1H)-pyrimidon-4 werden 30 h in 30 ml Pyridin am Rückfluß gekocht. Nach Abdestillieren des Pyridins wird der Rückstand an $Al_2O_3$ neutral, Akt. St. III, Laufmittel Methylenchlorid/3 % Methanol, chromatographiert. Das erhaltene Öl wird mit Weinsäure in Isopropanol zu einem amorphen Feststoff umgesetzt.

$C_{23}H_{31}N_7O_2$ • 0,5 Weinsäure $H_2O$

Ber.: C 51,1 % H 6,2 % N 14,4 %

Gef.: C 51,3 % H 6,5 % N 14,4 %

NMR (DMSO-$D_6$): 8,31 s [1]; 8,00 s [1]; 7,45 -7,55 m [2]; 7,20 -7,37 m [1]; 7,12 d [1]; 4,29 t [2]; 4,15 - (Weinsäure); 3,87 s [2]; 3,46 s [2]; 3,28 -3,40 m - [2]; 2,68 -2,90 m [4]; 2,35 s [3]; 1,82 t [2]; 1,35 - 1,70 m [6].

Beispiel 2

1-Methyl-3-amino-5-[3-(4-piperidinomethylpyrazol-1-oxy)propylamino]-1,2,4-triazol

5 g 3-(4-Piperidinomethylpyrazol-1-oxy)-propylamin und 4,9 g N-Cyano-1-methyl-2-(phenylmethylen)-hydrazincarboximidothiosäuremethylester werden zusammengegeben und 1 h auf 70 °C erwärmt. Anschließend wird das Reaktionsgemisch mit 40 ml 1 n Salzsäure aufgenommen, 1 h bei Raumtemperatur gerührt, mit Natronlauge alkalisch gestellt und mit Methylenchlorid extrahiert. Der nach Vertreiben des Lösungsmittels verbleibende Rückstand wird an $Al_2O_3$ neutral, Akt. St. III, Laufmittel Methylenchlorid/3 % Methanol chromatographiert. Die ölige Titelverbindung wird mit Weinsäure in Isopropanol zu einem amorphen Feststoff umgesetzt.

$C_{15}H_{26}N_8O_4$ • 0,5 Weinsäure • $H_2O$

Ber.: C 43,6 % H 6,4 % N 15,4 %

Gef.: C 44,0 % H 6,7 % N 19,3 %

NMR (DMSO-$D_6$): 7,93 s [1]; 7,31 s [1]; 4,29 t [2]; 4,12 (Weinsäure; 3,85 s [2]; 3,22 s breit [2 + 3]; 2,70 -2,88 m [4]; 1,82 t [2]; 1,35 -1,70 m [6].

In analoger Weise wurden hergestellt:

Beispiel 3

1-Methyl-3-amino-5-[4-(4-piperidinomethylpyrazol-1-oxy)butylamino]-1,2,4-triazol

$C_{16}H_{28}N_8O$ • 2 Oxalsäure • $H_2O$

Ber.: C 43,9 % H 5,8 % N 20,5 %

Gef.: C 43,7 % H 5,8 % N 19,7 %

NMR (DMSO-$D_6$): 7,91 s [1]; 7,38 s [1]; 4,27 t [2]; 4,03 s [2]; 3,28 s [3]; 3,15 - 3,25 m [2]; 2,65 -2,90 m [4]; 1,55 -1,75 m [10].

Beispiel 4

1-Methyl-3-amino-5[5-(4-piperidinomethylpyrazol-1-oxy)pentylamino]-1,2,4-triazol

Fp.: 68 -71 °C

$C_{17}H_{30}N_8O$ •0,5 $H_2O$

Ber.: C 55,7 % H 8,3 % N 30,6 %

Gef.: C 55,5 % H 8,2 % N 30,4 %

NMR (DMSO-$D_6$): 7,65 s [1]; 7,16 s [1]; 4,22 t [2]; 3,38 s [2]; 3,26 s [3]; 3,09 -3,18 m [2]; 2,19 -2,37 m [4]; 1,28 -1,70 m [12].

Beispiel 5

3-Amino-4-[4-(4-piperidinomethylpyrazol-1-oxy)-butylamino]-cyclobutendion-1,2

2,5 g 4-(4-Piperidinomethylpyrazol-1-oxy)-butylamin und 1,2 g 3-Amino-4-methoxy-cyclobutendion-1,2 werden in 80 ml Methanol gegeben und 12 h bei 20 °c gerührt. Anschließend wird der gebildete Feststoff abgesaugt.

Fp.: 231 °C

$C_{17}H_{25}N_5O_3$

Ber.: C 58,7 % H 7,3 % N 20,2 %

Gef.: C 58,3 % H 7,1 % N 20,2 %

NMR (DMSO-$D_6$): 7,62 s [1]; 7,14 s [1]; 4,27 t [2]-;3,38 s [2]; 3,10 -3,15 m [2]; 2,20 -2,40 m [4]; 1,29 -1,80 m [10].

In analoger Weise wurde hergestellt:

Beispiel 6

3-Methylamino-4-[4-(4-piperidinomethylpyrazol-1-oxy)butylamino]-cyclobutendion-1,2

Fp.: 178 -181 °C

$C_{18}H_{27}N_5O_3$

Ber.: C 59,8 % H 7,5 % N 19,4 %

Gef.: C 59,4 % H 7,5 % N 19,5 %

NMR (DMSODd$_6$): 7,66 s [1]; 7,12 s [1]; 4,25 t [3]; 3,39 s [2]; 3,28 s [3]; 3,10 - 3,17 m [2]; 2,22 -2,33 m [4]; 1,30 -1,75 m [10].

Beispiel 7

4-Methylamino-3-[3-(4-piperidinomethylpyrazol-1-oxy)propylamino]-1,2,5-thiadiazol-1-oxid

2,4 g 4-Methylamino-3-methoxy-1,2,5-thiadiazol-1-oxid und 3,5 g 3-(4-Piperidinomethylpyrazol-1-oxy)propylamin werden n 50 ml Methanol gelöst und 12 h bei Raumtemperatur stehen gelassen. Anschließend wird eingeengt und der Rückstand an $Al_2O_3$ neutral, Akt. St. III, Laufmittel Methylenchlorid/3 % Methanol, chromatographiert. Es werden 4 g der Titelverbindung erhalten, die aus Essigester kristallisiert wird.

Fp.: 106 °C

$C_{15}H_{25}N_7O_2S$

Ber.: C 49,0 % H 6,5 % N 26,7 % S 8,7 %

Gef.: C 48,4 % H 6,7 % N 26,6 % S 8,8 %

NMR (DMSO-D$_6$): 7,70 s [1]; 7,15 s [1]; 4,30 t [2]; 3,41 -3,60 m [2]; 3,32 s [2]; 2,90 d [3]; 2,20 -2,38 m [4]; 1,85 -2,00 [2]; 1,28 -1,58 m [6].

In analoger Weise wurden die Beispiele 8 bis 16 hergestellt:

Beispiel 8

4-Amino-3[3-(4-piperidinomethylpyrazol-1-oxy)-propylamino]-1,2,5-thiadiazol-1-oxid

$C_{14}H_{23}N_7O_2S \bullet 0,5\ H_2O$

Ber.: C 46,4 % H 6,6 % N 27,1 %

Gef.: C 46,0 % H 6,4 % N 27,0 %

NMR (DMSO-D$_6$): 7,76 s [1]; 7,18 s [1]; 4,32 t [2]; 2,44 -3,56 m [2]; 3,35 s [2]; 2,28 -2,45 m [4]; 1,86 - 2,00 m [2]; 1,32 -1,56 m [6].

Beispiel 9

4-Amino-3[3(4-N,N-dimethylaminomethylpyrazol-1-oxy)propylamino]-1,2,5-thiadiazol-1-oxid

$C_{11}H_{19}N_7SO_2 \bullet 0,7\ H_2O$

Ber.: C 40,6 % H 6,3 % N 30,1 % S 9,8 %

Gef.: C 41,4 % H 6,3 % N 29,8 % S 9,6 %

NMR (DMSO-D): 7,74 s [1]; 7,17 s [1]; 4,34 t [2]; 3,45 -3,56 m [2]; 3,28 s [2]; 2,12 s [6]; 1,90 -2,00 m [2].

Beispiel 10

4-Amino-3[4-(4-piperidinomethylpyrazol-1-oxy)-butylamino]-1,2,5-thiadiazol-1-oxid

Fp.: 135 -138 °C

NMR (DMSO-D$_6$): 7,65 s [1]; 7,15 s [1]; 4,23 t (2); 3,35 -3,50 m [2]; 3,30 s [2]; 2,20 -2,40 m [4]; 1,60 -1,80 m [4]; 1,25 -1,55 m [6].

Beispiel 11

4-Amino-3[5-(4-piperidinomethylpyrazol-1-oxy)-pentylamino]-1,2,5-thiadiazol-1-oxid

Fp.: 125 -127 °C

$C_{16}H_{27}N_7O_2S$

Ber.: C 50,3 % H 7,1 % N 25,7 % S 8,4 %

Gef.: C 49,8 % H 7,2 % N 25,3 % S 8,3 %

NMR (DMSO-$D_6$): 7,65 s [1]; 7,15 s [1]; 4,25 t [2]; 3,25 -3,45 m [4]; 2,20 -2,40 m [4]; 1,55 -1,70 m - [4]; 1,30 -1,50 m [8].

Beispiel 12

4-Methylamino-3[4-(4-piperidinomethylpyrazol-1-oxy)butylamino]-1,2,5-thiadiazol-1-oxid

$C_{16}H_{27}N_7O_2S$

Ber.: C 50,3 % H 7,1 % N 25,7 % S 8,4 %

Gef.: C 49,7 % H 7,3 % N 25,4 % S 8,4 %

NMR (DMSO-$D_6$): 7,65 s [1]; 7,15 s [1]; 4,25 t [2]; 3,35 -3,50 m [2]; 3,25 s [2]; 2,90 d [3]; 2,20 -2,40 m [4]; 1,60 -1,80 m [4]; 1,25 -1,55 m [6].

Beispiel 13

4-Amino-3[4-(4-morpholinomethylpyrazol-1-oxy)-butylamino]-1,2,5-thiadiazol-1-oxid

Fp.: 115 °C

$C_{14}H_{23}N_7SO_3$

Ber.: C 45,5 % H 6,3 % N 26,5 % S 8,7 %

Gef.: C 45,2 % H 6,3 % N 26,2 % S 8,6 %

NMR (DMSO-$D_6$): 7,65 s [1]; 7,15 s [1]; 4,25 t [2]; 3,45 -3,60 m [4]; 3,25 - 3,40 m [4]; 2,20 -2,40 m - [4]; 1,60 -1,80 m [4].

Beispiel 14

4-Amino-3[4-(4-pyrrolidinomethylpyrazol-1-oxy)-butylamino]-1,2,5-thiadiazol-1-oxid

Fp.: 100 °C (Zersetzung)

$C_{14}H_{23}N_7SO_2$

Ber.: C 47,6 % H 6,6 % N 27,7 % S 9,1 %

Gef.: C 47,2 % H 6,6 % N 27,2 % S 8,7 %

NMR (DMSO-$D_6$): 7,65 s [1]; 7,15 s [1]; 4,26 t 2,, 3,25 -3,60 m [4]; 2,35 -2,50 m [4]; 1,50 -1,80 m - [8].

Beispiel 15

4-Amino-3[4(4-N-methyl-N-benzylaminomethylpyrazol-1-oxy)butylamino]-1,2,5-thiadiazol-1-oxid

Fp.: 116 °C

$C_{18}H_{25}N_7SO_2$

Ber.: C 53,6 % H 6,8 % N 24,3 % S 7,9 %

Gef.: C 53,1 % H 6,4 % N 23,9 % S 7,8 %

NMR (DMSO-$D_6$): 7,75 s [1]; 7,22 -7,38 m [5]; 7,20 s [1]; 4,25 t [2]; 3,30 - 3,45 m [6]; 2,05 s [3]; 1,60 -1,80 m [4].

Beispiel 16

4-Amino-3[4-(4-N,N-di-n-butylaminomethylpyrazol-1-oxy)butylamino]-1,2,5-thiadiazol-1-oxid

Fo.: 115 °C

$C_{18}H_{33}N_7O_2$

Ber.: C 52,5 % H 8,1 % N 23,8 % S 7,8 %

Gef.: C 51,9 % H 8,1 % N 23,3 % S 7,6 %

Beispiel 17

N-Methyl-N'-[3-(4-piperidinomethylpyrazol-1-oxy)-propylamino]-cyanguanidin

4,3 g 3-(4-Piperidinomethylpyrazol-1-oxy)-propylamin und 2,7 g Dimethylcyandithioimidocarbonat werden in 50 ml Methanol gelöst und 12 h bei Raumtemperatur stehen gelassen. Anschließend werden 12 ml 16prozentige methanolische Methylaminlösung zugefügt und 24 h bei 50 °C gehalten. Nach Abdampfen des Lösungsmittels wird über $Al_2O_3$ neutral, Akt. St. III, Laufmittel Methylenchlorid, chromatographiert.

Man erhält 4 g der Titelverbindung
$C_{15}H_{25}N_7O \bullet 0,5\ H_2O$

Ber.: C 54,1 % H 8,0 % N 29,5 %

Gef.: C 54,1 % H 7,9 % N 29,4 %

NMR (DMSO-$D_6$): 7,68 s [1]; 7,16 s [1]; 4,24 t [2]; 3,24 -3,38 m [2]; 3,29 s [2]; 2,65 -2,76 m [3]; 2,22 - 2,40 m [4]; 1,75 -1,92 m [2]; 1,30 -1,56 m [6].

Beispiel 18

1-Methylamino-1-[3-(4-N,N-dimethylaminomethylpyrazol-1-oxy)propylamino]-2-nitroethylen

3,1 g 3-(4-N,N-Dimethylaminomethylpyrazol-1-oxy)propylamin und 2,3 g 1-Methylthio-1-methylamino-2-nitroethylen werden in 30 ml Methanol gelöst und 12 h bei Raumtemperatur stehen gelassen. Anschließend wird eingeengt und der Rückstand über Aluminiumoxid neutral, Akt. St. III, Laufmittel Methylenchlorid -2 % Methanol, chromatographiert.

Man erhält 1,4 g der Titelverbindung als farbloses Öl.

$C_{12}H_{22}N_6O_3$ • 0,75 MeOH • 0,5 $H_2O$

Ber.: C 45,8 % H 7,8 % N 25,6 %

Gef.: C 45,9 % H 7,5 % N 25,7 %

NMR ($DMSO-D_6$): 7,75 s [1]; 7,20 s [1]; 6,45 -6,65 m [1]; 4,32 t [2]; 3,45 -3,55 m [2]; 3,28 s [2]; 2,72 - 2,95 m [3]; 2,12 s [6]; 1,84 -1,58 m [2].

Auf analoge Weise wurden hergestellt:

Beispiel 19

1-Methylamino-1-[3-(4-piperidinomethylpyrazol-1-oxy)propylamino]-2-nitroethylen

$C_{15}H_{26}N_6O_3$ • 0,5 $H_2O$ • 0,5 MeOH

Ber.: C 51,2 % H 8,0 % N 23,1 %

Gef.: C 51,3 % H 7,8 % N 23,4 %

NMR ($DMSO-D_6$): 7,72 s [1]; 7,16 s [1]; 6,52 s breit [1]; 4,30 t [2]; 3,46 -3,60 m [2]; 3,29 s [2]; 2,70 - 2,95 m [3]; 2,24 -2,38 m [4]; 1,82 -1,98 m [2]; 1,30 -1,55 m [6].

Beispiel 20

1-Methylamino-1-[4-(4-piperidinomethylpyrazol-1-oxy)butylamino]-2-nitroethylen

$C_{16}H_{28}N_6O_3$ • Weinsäure • i-PrOH • $H_2O$

Ber.: C 47,5 % H 7,6 % N 14,5 %

Gef.: C 47,9 % H 7,7 % N 14,4 %

NMR ($DMSO-D_6$): 7,91 s [1]; 7,37 s [1]; 6,50 s [1]; 4,32 t [2]; 4,15 (Weinsäure); 3,90 s [2]; 3,10 -3,30 m [2]; 2,65 -2,95 m [7]; 1,40 -1,75 m [10].

Beispiel 21

3-[4-(4-Piperidinomethylpyrazol-1-oxy)butylamino]-benzisothiazol-1,1-dioxid

3,5 g 4-(4-Piperidinomethylpyrazol-1-oxy)-butylamin und 1,4 g Triethylamin werden in 50 ml Methylenchlorid gelöst. Anschließend wird eine Lösung von 2,8 g 3-Chlorbenzisothiazol-1,1-dioxid in 15 ml Methylenchlorid zugetropft. Nach 3 h Stehen bei Raumtemperatur wird das Reaktionsgemisch mit Wasser gewaschen, die organische Phase eingeengt und der Rückstand an $Al_2O_3$ neutral, Akt. St. III, Laufmittel Methylenchlorid, chromatographiert. Man erhält 1,7 g der Titelverbindung, die aus Ether kristallisiert wird.

Fp.: 86 -89 °C

$C_{20}H_{27}N_5SO_3$ • 0,5 $H_2O$

Ber.: C 56,2 % H 6,6 % N 16,4 % S 7,5 %

Gef.: C 56,5 % H 6,6 % N 16,6 % S 7,6 %

NMR ($DMSO-D_66$): 8,15 -8,25 m [1]; 7,90 -8,00 m [1]; 7,75 -7,85 m [2]; 7,65 s [1]; 7,15 s [1]; 4,25 t - [2]; 3,50 m [2]; 3,22 s [2]; 2,10 -2,35 m [4]; 1,60 - 1,90 m [4]; 1,20 -1,55 m [6].

Beispiel 22

4-Amino-3[4-(4-piperidinomethylpyrazol-1-oxy)-butylamino]-1,2,5-thiadiazol

2,7 g 4-Amino-3[4-(4-piperidinomethylpyrazol-1-oxy)butylamino]thiadiazol-1-oxid werden in 70 ml Methanol gelöst und mit 6 ml konzentrierter Salzsäure versetzt. Nach 6 h Stehen bei 25 °C wird im Vakuum eingeengt, der Rückstand in 80 ml Methylenchlorid aufgenommen und mit 9 ml Triethylamin versetzt. Anschließend werden 2,4 g N,N-Thiobisphthalimid zugesetzt. Nach weiteren 3 h bei 25 °C wird mit 50 ml 2 n Kalilauge gewaschen und die organische Phase eingeengt. Der Rückstand wird an $Al_2O_3$ neutral, Akt. St. III, Laufmittel Methylenchlorid/2 % Methanol, chromatographiert. Man erhält 1,0 g Wertprodukt, das in Methanol mit Weinsäure in ein amorphes Salz überführt wird.

NMR (DMSO-$D_6$): 7,85 s [1]; 7,30 s [1]; 4,25 t - [2]; 4,10 (Weinsäure); 3,80 s [2]; 3,25 -3,35 m [2]; 2,70 -2,90 m [4]; 1,40 -1,75 m [10].

**Ansprüche**

1. 1,4-Disubstituierte Pyrazolderivate der allgemeinen Formel I

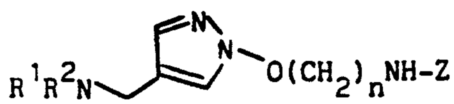 I,

worin $R^1$, $R^2$ jeweils unabhängig voneinander für Wasserstoff, Niedrigalkyl, Benzyl stehen oder zusammen mit dem Stickstoffatom, an das sie gebunden sind, ein Pyrrolidino-, Piperidino-oder Morpholino-Rest sein können, n eine ganze Zahl von 2 bis 5 ist, und Z für einen Rest der Formel

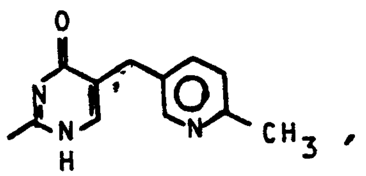 , 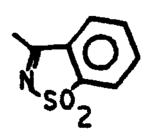 oder $QNR^1R^2$ steht,

wobei $R^1$, $R^2$ dasselbe wie oben bedeuten, Q für

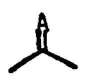 , 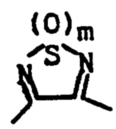 , 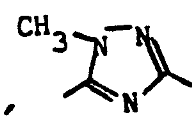 , 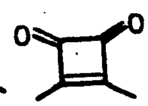 ,

A für $CHR^3$ oder $NR^3$ mit $R^3$ gleich CN, $NO_2$, $SO_2$Aryl, $SO_2$-Niedrigalkyl steht und m O oder 1 sein kann, sowie deren pharmazeutisch verträglichen Salze.

2. Verbindungen gemäß Anspruch 1, worin Z für

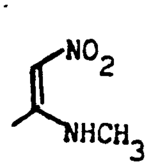 , 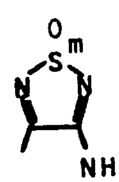 , 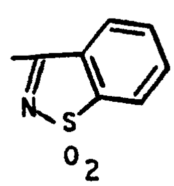 , 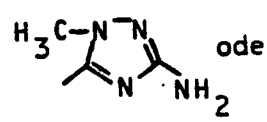 oder 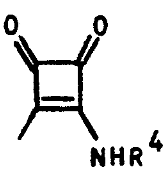

steht, wobei $R^4$ Wasserstoff oder Niedrigalkyl darstellt und m dasselbe wie oben bedeutet.

3. 4-Amino-3[4-(4-piperidinomethylpyrazol-1-oxy)butylamino]-1,2,5-thiadiazol-1-oxid.

4. 4-Methylamino-3[4-(4-piperidinomethylpyrazol-1-oxy)butylamino]-1,2,5-thiadiazol-1-oxid.

5. 4-Amino-3[4-(4-pyrrolidinomethylpyrazol-1-oxy)butylamino]-1,2,5-thiadiazol-1-oxid.

6. 4-Amino-3[4(4-piperidinomethylpyrazol-1-oxy)butylamino]-1,2,5-thiadiazol.

7. Verfahren zur Herstellung von Pyrazolderivaten der Formel I gemäß Anspruch I, dadurch gekennzeichnet, daß man

a) zur Herstellung von Verbindungen der Formel I mit Z gleich

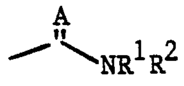

eine Thioverbindung der Formel II

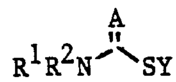 II,

in der R', R² und A dasselbe wie oben und Y Niedrigalkyl bedeuten, mit einem Amin der Formel III

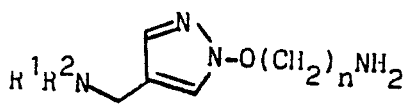 III,

in der R', R² und n die angegebene Bedeutung haben, umsetzt oder eine Verbindung der Formel III mit einer Verbindung der Formel IV oder V

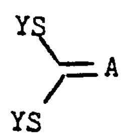 IV, 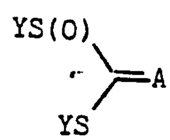 V,

in denen A und Y dasselbe wie oben bedeuten, umsetzt und die resultierende Verbindung der Formel VI

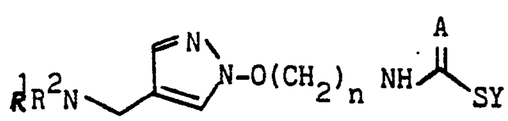 VI,

in der R', R², n und A die angegebene Bedeutung haben, mit einer Aminoverbindung der Formel VII

HNR'R² VII,

worin R', R² dasselbe wie oben bedeuten, reagieren läßt oder

b) zur Herstellung von Verbindungen der Formel I mit Z gleich

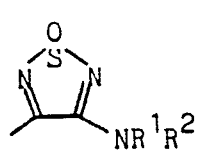

ein Thiadiazolderivat der Formel VIII

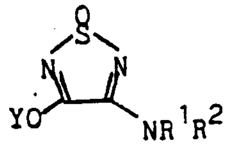

VIII,

in der R', R² und Y dasselbe wie oben bedeuten, mit einer Verbindung der Formel III umsetzt oder eine Verbindung der Formel III mit einer Verbindung der Formel IX

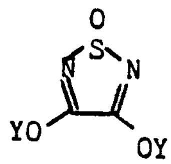

IX,

in der Y Niedrigalkyl bedeutet, umsetzt und die resultierende Verbindung der Formel X

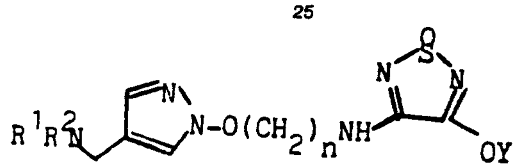

X

mit einer Aminoverbindung der Formel VII umsetzt oder

c) zur Herstellung von Verbindungen mit Z gleich

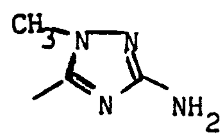

ein Hydrazinderivat der Formel XI

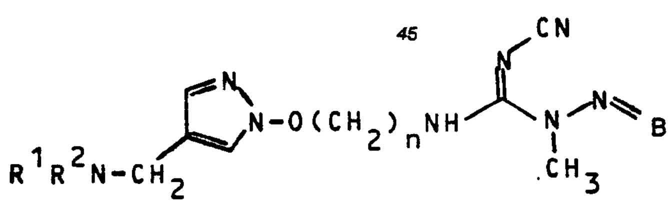

XI,

in der Y Niedrigalkyl und B eine Schutzgruppe, beispielsweise =CH-Ø, bedeuten, mit einer Verbindung der Formel III umsetzt und das Reaktionsprodukt in Gegenwart von Säuren katalytisch spaltet und cyclisiert oder

d) zur Herstellung von Verbindungen der Formel I mit Z gleich

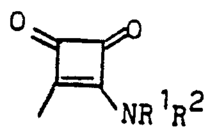

ein Quadratsäurederivat der Formel XII

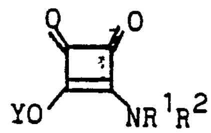

XII,

in der $R^1$, $R^2$ und Y dasselbe wie oben bedeuten, mit einer Verbindung der Formel III umsetzt oder ein Quadratsäurederivat der Formel XIII

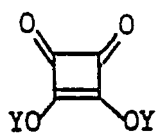

XIII,

in der Y (Niedrig)alkyl bedeutet, mit einer Verbindung der Formel III umsetzt und das resultierende Produkt der Formel XIV

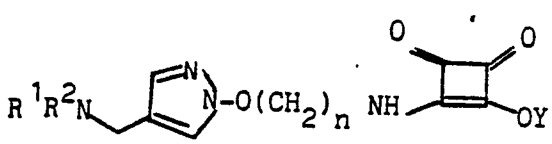

XIV,

in der $R^1$, $R^2$, Y und n dasselbe wie oben bedeuten, mit einem Amin der Formel VII umsetzt oder

e) zur Herstellung von Verbindungen der Formel I mit Z gleich

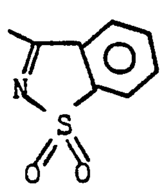

eine Verbindung der Formel XV

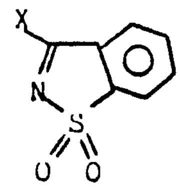

XV,

worin X eine geeignete Austrittsgruppe wie Halogen, OY, SY mit Y gleich Niedrigalkyl bedeutet,

mit einer Verbindung der Formel III umsetzt, oder

f) zur Herstellung von Verbindungen der Formel I mit Z gleich

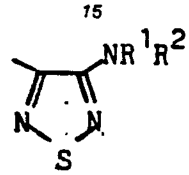

eine Verbindung der Formel I mit Z gleich

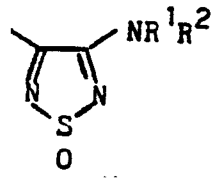

mit einer starken Säure behandelt und das Produkt der Formel XVI

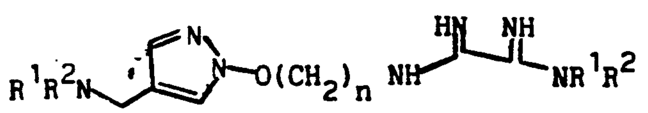

XVI

mit einer Verbindung der Formel XVII

L-S-L XVII,

in der L eine geeignete Austrittsgruppe, vorzugsweise Phthalimido-bedeutet, umsetzt,

und die so erhaltenen Verbindungen in ihre Salze mit physiologisch verträglichen Säuren überführt, oder

g) zur Herstellung von Verbindungen der Formel I mit Z gleich

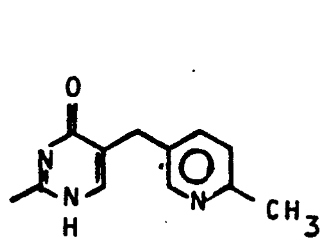

eine Verbindung der Formel III mit einer Verbindung der Formel

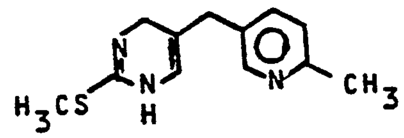

umsetzt.

8. Verbindungen der Formel III

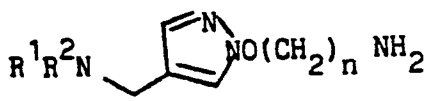 III,

worin $R^1$, $R^2$ und n dasselbe wie oben bedeuten.

9. Verbindungen der Formel I nach einem der Ansprüche 1 bis 6 oder eins ihrer physiologisch verträglichen Salze zur Verwendung als Arzneimittel.

10. Arzneimittel, das neben üblichen galenischen Hilfsmitteln eine Verbindung der Formel I nach einem der Ansprüche 1 bis 6 oder eins ihrer physiologisch verträglichen Salze als Wirkstoff enthält.

Europäisches Patentamt

# EUROPÄISCHER RECHERCHENBERICHT

Nummer der Anmeldung: EP 86112215.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A2 - 0 004 793 (SMITH KLINE)<br>* Anspruch 1 *<br>-- | 1 | C 07 D 401/12<br>C 07 D 417/12<br>C 07 D 401/14<br>C 07 D 231/08<br>C 07 D 403/12<br>A 61 K 31/415<br>A 61 K 31/41<br>A 61 K 31/425<br>A 61 K 31/505 |
| A | EP - A2,A3 - 0 117 345 (SMITH KLINE)<br>* Formel I; Seite 1, 2.-4. Absatz *<br>-- | 1,10 | |
| A | CHEMICAL ABSTRACTS, Band 99, Nr. 1, 4. Juli 1983, Columbus, Ohio, USA<br>TALIPOWA, M.A.; KAMILOW, A.I.; KHIDOYATOV, A.A.; KAMILOVA, R.M; MAKHSUMOW, A.G.; USMANOV, Kh.U.; " Results of the testing of pyrazolyl benzoates and some of their substituted derivatives for herbicidal activity"<br>Seite 185, Spalte 2, Zusammenfassung-Nr. 1 737b<br>& Dokl. Akad. Nauk Uzb. SSR 1982, (12), 31-2<br>-- | 1 | **RECHERCHIERTE SACHGEBIETE (Int. Cl.4)**<br>C 07 D 401/00<br>C 07 D 403/00<br>C 07 D 417/00 |
| A | US - A - 4 330 544 (JARREAU)<br>* Formel I *<br>---- | 1 | |

Der vorliegende Recherchenbericht wurde für alle Patentansprüche erstellt.

| Recherchenort | Abschlußdatum der Recherche | Prüfer |
|---|---|---|
| WIEN | 12-12-1986 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument